**Europäisches Patentamt**

**European Patent Office**

**Office européen des brevets**

(19)

(11) Publication number : **0 533 641 A2**

## (12) EUROPEAN PATENT APPLICATION

(21) Application number : **92850174.1**

(22) Date of filing : **15.07.92**

(51) Int. Cl.$^5$ : **A61L 25/00, C09K 3/10**

(30) Priority : **19.08.91 US 747295**

(43) Date of publication of application :
**24.03.93 Bulletin 93/12**

(84) Designated Contracting States :
**AT BE CH DE DK ES FR GB GR IT LI LU MC NL PT SE**

(71) Applicant : **Smith, Novis W. Jr.**
**412 South Perth Street**
**Philadelphia, PA 19147 (US)**

(72) Inventor : **Smith, Novis W. Jr.**
**412 South Perth Street**
**Philadelphia, PA 19147 (US)**

(74) Representative : **Onn, Thorsten et al**
**AB STOCKHOLMS PATENTBYRA, Zacco &**
**Bruhn, Box 23101**
**S-104 35 Stockholm (SE)**

(54) **Coating composition for parts.**

(57)   A method for forming a seal or coating with a composition comprising polyvinylidene difluoride and an aqueous emulsion of acrylates, methacrylates, an unsaturated carboxylic acid and/or acrylamide and a solvent having a low boiling point. The composition can be used to form a protective coating on parts or to form seals with joints or to form a bandage on mammals.

EP 0 533 641 A2

Field of the Invention

The present invention relates to novel quick coating compositions for providing protective coatings, especially spray-on coatings for human and veterinary use, and plumbing parts and joints to prevent leakage. The coatings can be used to form bandages on mammals, repair and/or seal protective garments and equipment from chemical agents.

Background of the Invention

In the plumbing field it is common to use joint compound and Teflon thread on plumbing connections to ensure a leak-free connection. The joint compound is awkward to handle and the Teflon thread is difficult to use and to form a uniform coating over pipe threads. Moreover, the layman does not have the proper technique to create consistently good seals without trial and error. In the electronics field, parts should be dust free and protected from contamination prior to installation to ensure quality performance. It is common practice to now enclose electronic parts in protective plastic envelopes which are static free.

U.S. Patent No. 3,557,516 to Gould et al discloses a spray-on coating comprised of acrylates, methacrylates and acrylamides for similar use on humans and for veterinary use as the present composition. However, the coating formed does not provide good adhesion or flexibility.

It is further desirable to have transparent coatings for parts which are water proof, non-static and can be easily applied or removed. In the case of medical parts it is desirable that the coatings maintain sterility so that the part can be utilized immediately without sterilization. It is further advantageous if the coating itself was able to sterilize the part.

SUMMARY OF THE INVENTION

The present invention relates to a novel coating composition which can provide a rapidly drying coating for use as protection for mammals or inanimate objects.

In accordance with one embodiment of the invention, there is provided a coating composition which can rapidly form a transparent coating. The coating composition which is formed with some of the solvents and/or additives of the invention results in a sterile contamination-free coating.

In accordance with another embodiment of the invention, there is provided a coating composition which can be sprayed-on or otherwise applied to the parts to provide a protective coating against water or chemical agents or as a bandage or as gloves.

The spray-on coating composition containing sterilizing agents, such as iodophors can be utilized to act as a bandage or to coat parts, for example, medical devices and instruments to maintain sterility of the parts prior to use. While acetone is effective to sterilize on contact, the presence of a sterilizing agent such as an iodophor results in extended sterilization even when a tear may occur.

The coating composition can also waterproof labels, form seals such as on jars or bottles, and repair plastic garments, such as protective clothing.

According to another embodiment of the present invention, there is provided a coating composition for use by plumbers when making connections.

The compositions of the invention comprises about 50 to 90% by weight of polymer solids selected from the group consisting of polyvinylidene difluoride and/or the copolymers thereof, about 10 to 45% by weight of polymer solids of a member selected from the group consisting of a lower alkyl acrylate, a lower alkyl methacrylate, a hydroxy lower alkyl acrylate, an alpha, beta-unsaturated carboxylic acid having an acid number of about 20 to 150, acrylamide, N-lower acrylamide and the copolymers thereof, and a solvent having a low boiling point, preferably less than about 80° C, which can completely dissolve the components.

A preferred composition comprises:

a) about 50 to 90% by weight of solids of a polymer selected from the group consisting of polyvinylidene difluoride and the copolymers thereof.

b) about 10 to 45% by weight of polymer solids of an alpha, beta-unsaturated carboxylic acid having an acid number of about 20 to 150, and

c) a polymer selected from the group consisting of lower alkyl acrylate, a lower alkyl methacrylate, acrylamide, an N-lower alkyl acrylamide and the copolymers thereof, and a low boiling solvent in a sufficient amount to practically and rapidly form a coating.

Preferably the composition includes water. More preferably, the composition is prepared by mixing acetone and the polyvinylidene difluoride and/or the copolymers thereof, with an aqueous emulsion of the acrylic compounds. A solution/emulsion having a solids content of about 10 to 30% by weight has been found to be suitable for most coatings. Greater dilution would result in thinner coatings or gloves which can be built up in thickness by repeated application. A greater solids content results in a thicker coating which may require heat for curing.

The composition of the invention can be formed utilizing a polyvinylidene difluoride homopolymer or a copolymer thereof. However, the amount of the copolymer utilized should not be an amount which will effect the characteristics of the polyvinylidene difluoride. Up to about 15% of a copolymer such as polyethylene, methymlethacrylate or an acrylate have been found suitable for use in the invention.

Useful lower alkyl acrylates and methacrylates comprise those having 1 to 8 carbon atoms in the alkyl group such as methyl acrylate, ethyl acrylate, isopropyl acrylate, methyl methacrylate, propyl methacrylate, ethoxyethyl acrylate, methoxyethyl acrylate, methoxyethyl methacrylate, ethoxyethyl methacrylate, acrylamide, methacrylamide, N-alkyl substituted acrylamides and methacrylamides such as N-methyl methacrylamide and N-isopropyl methacrylamide and N-vinyl pyrrolidone. Also polymers of the alkyl acrylates and methacrylates with 30-50% hydroxyethyl and hydroxy propyl acrylates and methacrylates are included in this category.

In addition to the acrylic monomer and acrylic monomer mixtures set forth above to prepare polymers there can be added to the monomer or monomer mixture up to about 15% by weight of the total composition acrylic monomers of acrylic acid, methacrylic acid, itaconic acid, maleic acid and fumaric acid.

Preferred alkyl acrylates which can be used to prepared the acrylic polymer dispersant are ethyl acrylate, propyl acrylate, isopropyl acrylate, butyl acrylate, tertiary butyl acrylate, pentyl acrylate, hexyl acrylate, octyl acrylate, nonyl acrylate, decyl acrylate, lauryl acrylate and the like.

To prepare the composition of the invention it is advantageous to prepare an aqueous emulsion of the acrylic compounds prior to admixture with the polyvinylidene difluoride.

One particular useful and preferred aqueous acrylic composition in which dispersions of the acrylic polymer dispersant are used contains the following film-forming constituents:

An acrylic polymer of styrene or methyl methacrylate or a mixture of styrene and methyl methacrylate, a hydroxyalkyl acrylate or a hydroxyalkyl methacrylate and an unsaturated carboxylic acid such as acrylic acid or methacrylic acid in which the polymer had an acid number of about 20-150.

Other preferred acrylic polymer dispersant include the following:

1. methyl methacrylate, butyl acrylate and acrylic acid
2. isodecyl methacrylate, butyl acrylate and acrylic acid
3. stearyl methacrylate, butyl acrylate and acrylic acid
4. methyl methacrylate, 2-ethylhexyl acrylate and methacrylic acid.

Another preferred acrylic polymer dispersant which gives high quality dispersion is of styrene, methyl methacrylate, butyl acrylate and acrylic acid.

A preferred coating for parts comprises about 75 to 90% by weight of polyvinyldine difluoride, and 10 to 25% by weight of a mixture of methyl methacrylate, methyl methacrylate copolymer, methyl acrylate and acrylic acid and a plasticizer.

To make the coating opaque there may be added a clay, pigment, dye or the like.

The solvent utilized in the invention are those solvents or a mixture of solvents having a low boiling point, preferably less than 80° C which completely dissolves the components and quickly evaporates to form a coating. It is essential to achieve complete dissolution to obtain a spray which will not clog the spraying apparatus.

Typical solvents that can be used in the dispersion/solution process are diacetone alcohol, acetone, acetylacetone, cyclohexanone, ethylene glycol monoethylether, ethylene glycol monobutyl ether, ethylene glycol monomethylether acetate, tetrahydrofuran and the like. Solvents of limited water solubility can also be used such as methylethyl ketone, pentanone, tetrahydrofuran, ethylene glycol dimethyl ether; and the like.

Most preferable of the solvents is acetone since it is an excellent solvent and acts as a disinfectant.

It is a feature of the invention that the compositions of the invention can also be applied utilizing conventional means such as dipping, spraying, coating, etc.

Suitable propellants for use in the aerosols include those well known in the art. There can be used compressed gases such as dimethyl ether, carbon dioxide, nitrous oxide, nitrogen, air, liquified volatile hydrocarbons such as propane, N-butane, isobutane and 2-methylbutane, methylene chloride, vinyl chloride, fluorinated hydrocarbons such as dichlorodifluoromethane (Freon 12), trichlorofluoromethane, 1, 2-dichloro-1, 1-difluoroethane, vinyl fluoride, vinylidene difluoride, 1-chloro 1, 1 difluoroethane. The propellant should contain a substantial amount of volatile material boiling at not over 20 degrees Celsius, but there can also be present a significant amount of less volatile material boiling up to 50 degrees Celsius.

Plasticizers useful for film formation in combination with the polymer-powders include water soluble polar compounds including glycols such as propylene glycol, ethylene glycol, trimethylene glycol, 1,3-butanediol, 1,4-butanediol, 2,5-hexanediol, diethylene glycol, triethylene glycol, tetraethylene glycols and other polyethylene glycols and their methyl and ethyl ethers having a molecular weight up to 800 (e.g. hydroxy terminated polymers of ethylene or polypropylene oxide having average molecular weights of 200-800), dipropylene glycol, tripropylene glycol and other polypropylene glycols having molecular weights up to 900, propylene glycol monoethyl either, di(hydroxypropyl) oxalate, hydroxypropyl acetate, glyceryl tributyrate, liquid sorbitol ethylene oxide adducts, liquid glycerine ethylene oxide adducts, diethylene glycol monomethyl ether, diethylene glycol monoethyl ether, ethylene glycol diacetate.

The amount of plasticizer utilized depends upon the acrylic polymers utilized and the characteristics of the coating desired. About 5 to 10% by weight of plasticizer to solids content has been found suitable for most strippable coatings.

For use as a bandage or coating for mammals there may be incorporated into the coating composition disinfecting and/or sanitizing agents, for example, povidone iodine, antiinflammatory agents, such as steroids, and the like.

For forming a non-leak seal for plumbing parts, a composition of the invention can be sprayed on the threads of a part forming the connection and then threaded onto a mating part so as to set and form a leak-proof connection. Alternatively, sealing composition can be applied with a suitable applicator whereby a thicker coating would be applied that would be distributed upon threading to form a seal when set.

Small parts may be placed on a base substrate and spray coated so as to adhere to the base thereby forming a display and a protective environment. Such a procedure is advantageous for medical devices.

The following example illustrated the invention. The parts and percentages and ratios are on a weight basis unless otherwise specified.

## EXAMPLE 1

A composition suitable for dipping parts to form a coating was prepared as follows:

|  | WEIGHT |
| --- | --- |
| Kynar (polyvinylidene difluoride) | 24 g. |
| Aqueous acrylic polymer composition | 3 g. |

An aqueous composition containing 35% polymer solids in which the polymer solids are methyl methacrylate/butyl acrylate/hydroxyethyl acrylate/acrylic acid in a weight of about 20,000 determined by gel permeation chromatography using polystyrene as a standard and having 80% of the carboxyl groups neutralized with diethylethanolamine and deionized water.

|  | Wt. |
| --- | --- |
| Plasticizer | 1 g. |
| Acetone | 80 g. |

The ingredients were mixed and heated to a temperature of 50°C. The mixture formed a gel upon cooling which reversed by reheating. A medicament can be incorporated into the mixture or a suitable sanitizing or disinfecting agent or a medicament.

## EXAMPLE 2

A. A neutralized acrylic dispersant is prepared as follows:

|  | PARTS BY WEIGHT |
|---|---|
| Aqueous acrylic polymer Composition of Example 1 | 100 g. |
| Diethanol amine | 22 g. |
|  | 122 g. |

B. A sprayable composition was prepared as follows:

|  |  |
|---|---|
| Polyvinylidene difluoride | 22 g. |
| Methyl methacrylate copolymer | 2 g. |
| Dispersant from part A | 3 g. |
| Acetone | 80 g. |

The mixture was heated to 50°C to place the ingredients into solution and an additional 80 grams of acetone was added. The composition is then used in a container with an inert propellant.

## EXAMPLE 3

A series of experiments were run utilizing commercially available compositions. There was utilized Kynar, a polyvinylidene difluoride powder available from Pennwalt, Philadelphia, PA. Rhoplex B-15, an acrylic polymer having a glass transition temperature of 0°C, a powder available from Rohm & Haas, Philadelphia, PA, Acryloid A-11, a 30-40% solution of methyl methacrylate and acrylates having a glass transition temperature of 100°C, and acryloid B-44 or methyl methacrylate copolymer having a glass transition temperature of 60°C, each available from Rohm & Haas.

All were mixed together with 80 grams of acetone and heated to 50°C.

| RUN | KYNAR | ACRYLOID A-11 | PHOPLEX B-15 | ACRYLOID B-44 |
|---|---|---|---|---|
| A | 24 |  | 3 | 1 |
| B | 24 | 1 | 3 |  |
| C | 25 |  | 2 | 1 |
| D | 23 |  | 4 | 1 |

All of the compositions yielded a coating which was tough, resilient, notch resistant and flexible.

## Claims

1. A protective coating for mammals or parts which comprises:
   a) about 50 to 90% by weight of solids selected from the group consisting of polyvinylidene difluoride and the copolymers thereof,

b) about 10 to 45% by weight of solids of a member selected from the group consisting of a lower alkyl acrylate, a lower alkyl methacrylate, a hydroxy lower alkyl acrylate, a hydroxy lower alkyl acrylate, an alpha beta unsaturated carboxylic acid having an acid number of about 20 to 150, acrylamide, an N-lower alkyl acrylamide and the copolymers thereof, and a solvent having a low boiling point in a sufficient amount to completely dissolve all components.

2. The composition of claim 1 including water.

3. The composition of claim 1 wherein said solvent is acetone.

4. The composition of claim 1 wherein said solvent has a boiling point less than 80°C.

5. The composition of claim 1 comprising about 75 to 90% by weight of polyvinylidene difluoride and 10 to 25% by weight of methyl methacrylate, methyl methacrylate copolymer, methacrylate and acrylic acid.

6. The composition of claim 1 including a plasticizer.

7. The composition of claim 1 comprising about 10 to 30% by weight solids.

8. The composition of claim 1 which is sprayable.

9. The composition of claim 1 including a medicament.

10. The method of claim 1 where the coating is sprayed on electronic parts and circuit boards.

11. The combination of a part and a coating formed by the method of claim 1.

12. The combination of a part and a coating formed by the method of claim 2.

13. The combination of a part and a coating formed by the method claim 3.